# EUROPEAN PATENT SPECIFICATION

(11) **EP 3 742 170 B1**
(45) Date of publication and mention of the grant of the patent: **03.01.2024**
(21) Application number: 18901231.3
(22) Date of filing: 08.11.2018
(51) Int. Cl.: G01N 35/00, G01N 33/68, G01N 35/10, G01N 27/447, G01N 35/04

(54) **FULL-AUTOMATIC WESTERN BLOTTING ANALYZER**
VOLLAUTOMATISCHER WESTERN-BLOT-ANALYSATOR
ANALYSEUR DE TRANSFERT WESTERN ENTIÈREMENT AUTOMATIQUE

(30) Priority: 19.01.2018 CN 201810055656
(43) Date of publication of application: 25.11.2020
(73) Proprietor: Shenzhen Yhlo Biotech Co., Ltd, 518116 Shenzhen (CN)
(72) Inventor: HU, Kunhui, Shenzhen, Guangdong 518116 (CN); WU, Benqing, Shenzhen, Guangdong 518116 (CN); CHEN, Tianlong, Shenzhen, Guangdong 518116 (CN); LIN, Guodong, Shenzhen, Guangdong 518116 (CN); SONG, Yongbo, Shenzhen, Guangdong 518116 (CN)
(74) Representative: Nederlandsch Octrooibureau
(86) International application number: PCT/CN2018/114463
(87) International publication number: WO 2019/140988

(56) References cited:
- EP-A1- 0 787 990
- CN-A- 106 461 606
- CN-A- 108 120 840
- CN-U- 203 658 374
- CN-U- 203 658 374
- CN-U- 204 758 618
- CN-U- 205 176 039
- CN-U- 206 832 821

## Description

### TECHNICAL FIELD

The present disclosure relates to the technical field of detection devices, in particular to a full-automatic immunoblotting analyzer.

### BACKGROUND

Immunoblotting, also known as Western blotting, is a method for detecting a certain protein in complex samples based on a specific binding of an antigen and an antibody. It is a new immunological biochemical technology developed on the basis of gel electrophoresis and solid-phase immunoassay technology. Immunoblotting is often used to identify a certain protein, and can perform qualitative and semi-quantitative analysis of the protein. Combined with chemiluminescence detection, immunoblotting can compare the expression differences of the same protein in multiple samples at the same time. The immunoblotting method detects via a specific immune reaction between an antibody and an epitope presented by a target protein attached to a solid support. The whole process mainly includes: protein separation, electrotransfer, immunological detection and signal detection.

The detection method of the traditional immunoblotting analyzer is generally manual, and different reagents need to be replaced. Frequent operations increase the working intensity and working time of the experimenter, and the manual operations are tedious and complicated. There are many contacts between people and potentially infected items, thus the detection security is poor.

CN 203 658 374 U discloses an utility model relating to a kind of full-automatic immunoblotting assay instrument, belonging to medical treatment detection device technical field. CN 206 832 821 U discloses an utility model relating to an automatic western blot analyzer with a tilted incubator turntable, belonging to the technical field of an incubator turntable.

EP 0 787 990 A1 discloses an automatic analyzer comprising a sampler, a reaction table, a reagent table, a dispenser, a photometer, wherein reaction tubes disposed on the reaction table are rotated several times and moved by one pitch per revolution of the reaction table, photometry is effected synchronously, and the quantities of the components are measured.

### SUMMARY

Accordingly, the present disclosure provides a full-automatic immunoblotting analyzer, which does not require human involvement in the testing process, and the modules cooperate with each other to realize the whole process from sample feeding to result output.

A full-automatic immunoblotting analyzer includes:
a reagent module configured to load a reagent required for testing;
a sample feeding module configured to receive a sample and deliver the sample to a specified sampling station;
an adding module configured to suck the reagent from the reagent module and deliver the reagent to a specified reagent adding station, which is a testing slit of a incubating module; the adding module is also configured to suck the sample from the adding module and deliver the sample to a specified sample adding station, which is the testing slit of the incubating module;
a washing module configured to wash the adding module;
an incubating module configured to receive the reagent and the sample delivered by the adding module, and mix and incubate the reagent and the sample; the incubating module is provided with a testing slit configured to carry the reagent and the sample;
a liquid injection and suction module provided at an edge of the incubating module and configured to inject washing liquid and suck liquid from the incubating module;
a determining module provided at the edge of the incubating module and configured to take an image of a substance in the incubating module, and analyze the image analysis, and output a result; and
a drying module provided at the edge of the incubating module and configured to dry the incubating module.

In the aforementioned full-automatic immunoblotting analyzer, the incubating module is taken as a core, and the other modules are configured around the incubating module. According to the testing items, the sample feeding module, the reagent module, and the adding module are used to realize automatic sample feeding and automatic reagent adding, the washing module is used to wash the adding module to avoid contamination between the medicaments, the liquid injection and suction module is used to inject the washing liquid to the incubating module and discharge the waste liquid from the incubating module, the drying module is used to dry the incubating module, and the determining module is used to analyze the image of the incubating tray and output the result. The full-automatic immunoblotting analyzer does not require human involvement in the test process, and the modules cooperate with each other to realize the whole process from sample feeding to result output.

In one of the embodiments, the sample feeding module includes: a first X-axis movement component, a first Y-axis movement component connected to the first X-axis movement component, and a first Z-axis movement component connected to the first Y-axis movement component; the first X-axis movement component, the first Y-axis movement component, and the first Z-axis movement component cooperate with each other to realize a spatial three-dimensional action.

In one of the embodiments, the adding module includes a second Z-axis movement component and a horizontal θ-axis movement component connected to the second Z-axis movement component; the second Z-axis movement component is configured to adjust height, and the horizontal θ-axis movement component is configured to realize horizontal position adjustment via rotating and swinging.

In one of the embodiments, the incubating module includes a first driving component and a testing component connected to the first driving component; the first driving component includes a first driver and a main spindle connected to the first driver; the first driver is configured to drive the main spindle to rotate; an angle between the main spindle and a vertical direction is θ and θ is an acute angle; the testing component includes a disc sleeved on the main spindle and an incubating tray located in the disc; the disc is provided with a through hole allowing the main spindle to extend through; the disc is provided with a heat preserving pot to accommodate the incubating tray and a heating film connected to the thermal insulation pot; an angle between the incubating tray and a horizontal plane is β and β=θ; the incubating tray is connected to the main spindle and rotates with the main spindle; the incubating tray is provided with the testing slit; the testing slit is configured to carry the sample and the reagent.

In one of the embodiments, the disc is further provided with a pressing plate and a thermal insulation cotton component; the pressing plate is located at a bottom of the thermal insulation pot, and the heating film is located between the thermal insulation pot and the pressing plate; the thermal insulation cotton component is located outside the thermal insulation pot to keep the thermal insulation pot warm.

In one of the embodiments, the liquid injection and suction module includes: a frame, a liquid injecting component connected to the frame, and a liquid suction component connected to the frame; the liquid injection component includes a liquid injection fixing base hinged on the frame and a liquid injection needle connected to the liquid injection fixing base; the liquid suction component includes a liquid suction fixing base slidably connected to the frame and a liquid suction unit connected to the liquid suction fixing base; the liquid suction unit is provided with a liquid suction needle.

In one of the embodiments, the determining module includes: a camera facing the incubating tray, a light source disposed adjacent to the camera, and a determining component electrically connected to the camera; the determining component is configured to analyze the image and output the result.

In one of the embodiments, the drying module includes: a heater facing the incubating tray, a fan disposed adjacent to the heater, and a temperature sensor configured to detect a heating temperature.

In one of the embodiments, the full-automatic immunoblotting analyzer further includes: a box configured to accommodate the reagent module, the sample feeding module, the adding module, the washing module, the incubating module, the liquid injection and suction module, the determining module, and the drying module.

Meanwhile, a full-automatic immunoblotting analysis testing method is also provided.

A full-automatic immunoblotting analysis testing method includes:
providing a full-automatic immunoblotting analyzer, which includes: a reagent module, a sample feeding module, an adding module, a washing module, an incubating module, a liquid injection and suction module, a determining module, and a drying module; the reagent module is configured to load a reagent required for testing; the sample feeding module is configured to receive a sample and deliver the sample to a specified sampling station; the adding module is configured to suck the reagent from the reagent module and deliver the reagent to a specified reagent adding station, which is a testing slit of a incubating module; the adding module is also configured to suck the sample from the adding module and deliver the sample to a specified sample adding station, which is the testing slit of the incubating module; the washing module is configured to wash the adding module; the incubating module is configured to receive the reagent and the sample delivered from the adding module, and the reagent and the sample are mixed and incubated; the incubating module is provided with a testing slit configured to carry the reagent and the sample; the liquid injection and suction module is provided at an edge of the incubating module and configured to inject washing liquid and suck liquid from the incubating module; the determining module is disposed at the edge of the incubating module for image shooting, image analysis, and result output of the substance in the incubating module, the drying module is provided at the edge of the incubating module and configured to dry the incubating module;
sucking, by the adding module, amount of reagents from the reagent module and sucking amount of samples from the sample feeding module, and delivering, by the adding module, the reagents and the samples into the testing slit of the incubating module to perform an incubation operation; the reagents have one or more types; and prior to sucking different reagents and samples, washing, by the washing module, the adding module;
rotating the incubating module and incubating for a certain period of time, then draining, by the liquid injection and suction module, the liquid in all of the testing slits one by one;
injecting, by the liquid injection and suction module, washing liquid into all of the testing slits one by one;
activating the drying module, and starting the incubating module to rotate for a predetermined time and then stopping;
activating the determining module to identify objects in all of the testing slits one by one; and
outputting analysis result by the determining module.

In the aforementioned full-automatic immunoblotting analysis testing method, the incubating module is taken as a core, and the other modules are configured around the incubating module. According to the testing items, the sample feeding module, the reagent module, and the adding module are used to realize automatic sample feeding and automatic reagent adding, the washing module is used to wash the adding module to avoid contamination between the medicaments, the liquid injection and suction module is used to inject washing liquid to the incubating module and discharge the waste liquid from the incubating module, the drying module is used to dry the incubating module, and the determining module is used to analyze the image of the incubating tray and output the result. The full-automatic immunoblotting analyzer does not require human involvement in the test process, and the modules cooperate with each other to realize the whole process from sample feeding to result output.

### BRIEF DESCRIPTION OF THE DRAWINGS

FIG. 1 is a schematic view of a full-automatic immunoblotting analyzer according to an embodiment of the present disclosure;
FIG. 2 is a top view of the full-automatic immunoblotting analyzer shown in FIG. 1 after removing a protective cover;
FIG. 3 is a side view of an incubating module in the full-automatic immunoblotting analyzer shown in FIG. 1;
FIG. 4 is a side sectional view of the incubating module shown in FIG. 3;
FIG. 5 is a schematic view of a liquid injection and suction module of the full-automatic immunoblotting analyzer shown in FIG. 1;
FIG. 6 is a schematic view of the liquid suction unit in the liquid injection and suction module shown in FIG. 5.

The meaning of each reference numeral in the drawing is:
10- full-automatic immunoblotting analyzer; 20-reagent module; 30-sample feeding module; 40-adding module; 50-washing module; 60-incubating module, 61-first driving component, 62-main spindle, 63-first driver, 64-driving pulley, 65-timing pulley, 66-timing belt, 67-protective housing, 68-bearing, 69-testing component, 610-disc, 611-incubating tray, 612-thermal insulation pot, 613-heating film, 614-testing slit, 615-connecting block, 616-pressing plate, 617-first thermal insulation cotton, 618-second thermal insulation cotton, 619-third thermal insulation cotton, 620-bottom plate, 621-support column; 70-liquid injection and suction module, 71-frame, 72-support vertical plate, 73-main fixing plate, 74-connecting plate, 75-guiding rail, 76-injection component, 77-injection fixing base, 78-liquid injection needle, 79-suction component, 710-suction fixing base, 711-suction unit, 712-suction needle, 713-suction plate, 714-elastic member, 715-support base, 716-baffle, 718-screw, 719-second driving component, 720-second driver, 721-driven pulley, 722-transmission unit, 723-position detecting unit; 80-determining module; 90-drying module; 100-box, 101-protective cover.

### DETAILED DESCRIPTION OF THE EMBODIMENTS

In order to facilitate the understanding of the present disclosure, the present disclosure will be described more completely hereinafter with reference to the related accompanying drawings. Preferable embodiments of the present disclosure are presented in the accompanying drawings. However, the present disclosure may be embodied in many different forms and is not limited to the embodiments described herein. Rather, these embodiments are provided so that the understanding of the disclosure of the present disclosure will be more thorough and complete.

It should be understood that when an element is defined as "fixed to" another element, it is either directly on an element or indirectly on an element with a mediating element. When an element is considered to be "connected" to another element, it can be directly connected to another element or indirectly connected to another element with a mediating element.

Unless defined otherwise, all technical and scientific terms used herein have the same meaning as commonly understood by people who are skill in the art to which the present disclosure belongs. The terms used herein in the specification of the present disclosure is only for the purpose of describing specific embodiments, and is not intended to limit the present disclosure.

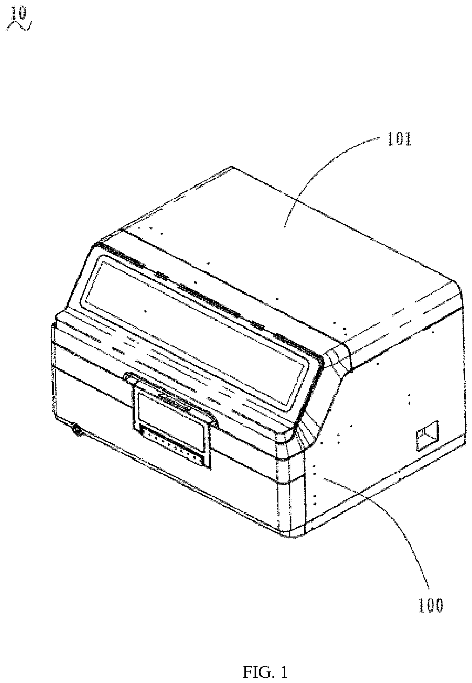

Referring to FIG. 1 to 6, which are schematic views of a full-automatic immunoblotting analyzer 10 according to a preferred embodiment of the present disclosure.

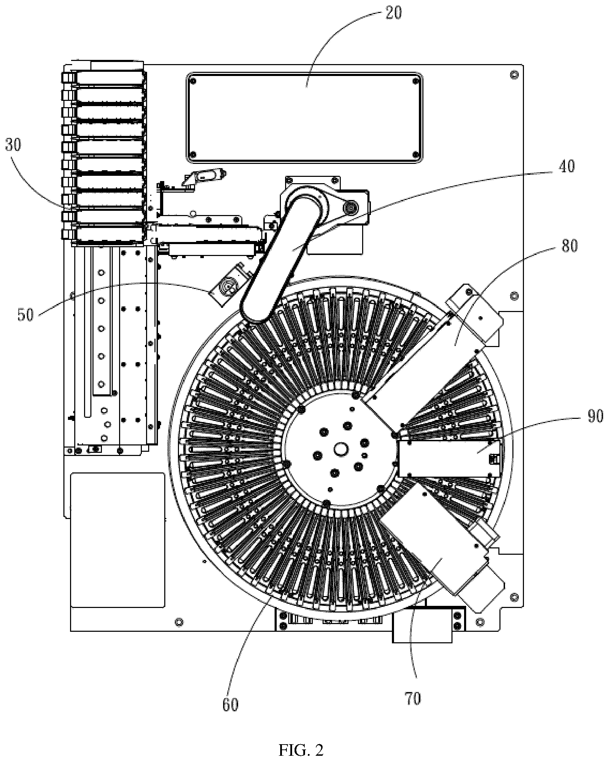

As shown in FIG. 1 and 2, a full-immunoblotting analyzer 10 includes: a reagent module 20, a sample feeding module 30, an adding module 40, a washing module 50, an incubating module 60, a liquid injection and suction module 70, a determining module 80, and a drying module 90. The reagent module 20 is configured to load a reagent required for testing. The sample feeding module 30 is configured to receive a sample and deliver the sample to a specified sampling station. The adding module 40 is configured to suck the reagent from the reagent module 20 and deliver the reagent to a specified reagent adding station, which is the incubating module 60. The adding module 40 is also configured to suck the sample from the adding module 40 and deliver the sample to a specified sample adding station, which is the incubating module 60. The washing module 50 is configured to wash the adding module 40. The incubating module 60 is configured to receive the reagent and the sample delivered by the adding module 40, and mix and incubate the reagent and the sample. The liquid injection and suction module 70 is provided at an edge of the incubating module 60 and is configured to inject washing liquid and suck liquid from the incubating module 60. The determining module 80 is provided at the edge of the incubating module 60 configured to take an image of a substance in the incubating module 60 and analyze the image, and output the result. The drying module 90 is provided at the edge of the incubating module 60 and configured to dry the incubating module 60. Each module takes the incubating module 60 as the core and is arranged around it, such that the layout between the modules is compact and reasonable. The following describes the structure of each module in this embodiment:

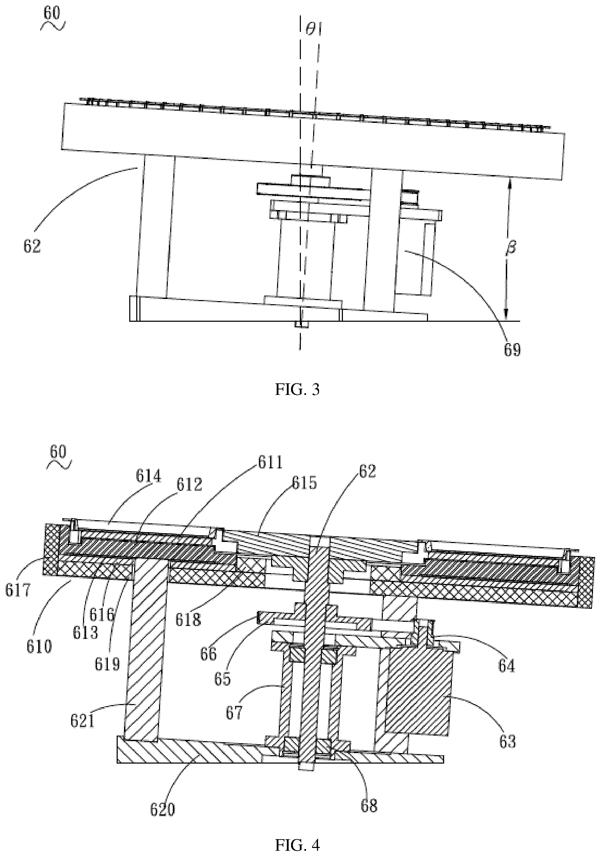

The incubating module 60 includes, as shown in FIG. 4: a bottom plate 620, a top plate arranged in parallel with and spaced apart above the bottom plate 620, and a pillar connected between the bottom plate 620 and the top plate. The top plate is provided with reagent grooves distributed in an array for holding various reagents.

The sample feeding module 30 includes: a first X-axis movement component, a first Y-axis movement component connected to the first X-axis movement component, and a first Z-axis movement component connected to the first Y-axis movement component. The first X-axis movement component, the first Y-axis movement component, and the first Z-axis movement component cooperate with each other to realize a spatial three-dimensional action.

The adding module 40 includes a second Z-axis movement component and a horizontal θ-axis movement component connected to the second Z-axis movement component. The second Z-axis movement component is configured to adjust height, and the horizontal θ-axis movement component is configured to realize horizontal position adjustment via rotating and swinging.

As shown in FIG. 3 and 4, the incubating module 60 includes a first driving component and a testing component 69 connected to the first driving component. The testing component 69 is configured to carry the substance to be tested to conduct heating, mixing and incubation, and the first driving component is configured to drive the testing component 69 to operate. In this embodiment, the first driving component includes a first driver 63 and a main spindle 62 connected to the first driver 63. The first driver 63 is configured to drive the main spindle 62 to rotate. An angle between the main spindle 62 and a vertical direction is θ and θ is an acute angle. In this embodiment, the first driver 63 is a motor. The first driver 63 and the main spindle 62 can be connected in multiple ways. For example, a rotor of the first driver 63 is connected to the main spindle 62 and is on the same axis as the main spindle 62. However, this solution will make the first driver 63 and the main spindle 62 accumulates the space in the height direction of the entire device, resulting in an increase in the height of the entire device. Therefore, an alternative implementation can also be adopted. For example, the first driver 63 is arranged in parallel with the main spindle 62, and a transmission between them can be performed by a transmission member connected between the first driver 63 and the main spindle 62, which can reduce a space in the height direction of the entire device after combining the first driver 63 and the main spindle 62. The transmission member may be a combination of transmission gears, or as shown in this embodiment: the transmission member includes: a drive pulley 64 sleeved on the rotor of the first driver 63, a synchronous pulley 65 sleeved on the main spindle 62, and a timing belt 66 coupled between the driving pulley 64 and the timing pulley 65. The first driver 72063 is located at one side of the main spindle 62, and the rotor of the first driver 63 is arranged in parallel with the main spindle 62.

The testing component 69 includes a disc 610 sleeved on the main spindle 62 and an incubating tray 611 embedded in the disc 610. The disc 610 is provided with a through hole allowing the main spindle 62 to extend through. The disc 610 is provided with a thermal insulation pot 612 configured to accommodate the incubating tray 611 and a heating film 613 connected to the thermal insulation pot 612. An angle between the incubating tray 611 and a horizontal plane is β and β=θ. The disc 610 does not rotate with the main spindle 62, while the incubating tray 611 is connected to the main spindle 62 and rotate with the main spindle 62. The incubating tray 611 is provided with testing slits 614. The incubating tray 611 is arranged in a circular ring structure and an outer diameter thereof is less than the radius of the disc 610. The incubating tray 611 is provided with a plurality of station tanks configured to accommodate the testing slits 614 (for example, in this embodiment, sixty station tanks are provided, and each station tank corresponds and accommodates a testing slit 614). The station tanks are arranged along a radial direction of the incubating tray 611 and are evenly distributed on the incubating tray 611. The station tanks can accommodate a batch of testing slits 614 during testing, and have a compact and efficient structure. In this embodiment, in order to facilitate the assembly, a connecting block 615 may be further provided on the testing component 69 to connect the incubating tray 611 and the main spindle 62. In other embodiments, the shape of the incubating tray 611 can also be adjusted to directly connect the main spindle 62. Some auxiliary equipment can also be provided to enhance the automation of the device. For example, the testing component 69 further includes a temperature sensor connected to the thermal insulation pot 612 and a temperature switch electrically connected to the temperature sensor. The temperature switch is electrically connected to the heating film 613. When the temperature sensor detects that the temperature of the thermal insulation pot 612 reaches the target temperature, the temperature switch turns off the heating film 613. It should be noted that when the incubating tray 611 is in operation, its rotation speed is controlled within the preset speed range, so as to ensure that the substance in the testing slit 614 will not stay in the end of testing slit 614 away from the main spindle 62 due to the centrifugal force, and ensure that the substance inside the testing slit 614 can reciprocate with the rotation of the incubating tray 611 to achieve the purpose of even mixing.

In this embodiment, in order to improve the temperature stability during incubation, the disc 610 may also be provided with a pressing plate 616 and a thermal insulation cotton component. The pressing plate 616 is located at a bottom of the thermal insulation pot 612, and the heating film 613 is located between the thermal insulation pot 612 and the pressing plate 616. The thermal insulation cotton component is located outside the thermal insulation pot 612 to keep the thermal insulation pot 612 warm. The thermal insulation cotton component is configured to enhance the thermal insulation effect of the thermal insulation pot 612, and the pressing plate 616 is configured to isolate the heating film 613 from the thermal insulation cotton component. In this embodiment, the thermal insulation cotton component includes: a first thermal insulation cotton 617 surrounding a side of the thermal insulation pot 612 away from the main spindle 62, a second thermal insulation cotton 618 surrounding a side of the thermal insulation pot 612 close to the main spindle 62, and a third thermal insulation cotton 619 laid on a bottom of the pressing plate 616.

In addition, the testing component 69 may further include a bottom plate 620 pivotally connected to the main spindle 62 and a support column 621 connected to the bottom plate 620 at one end. A loading surface of the bottom plate 620 is an inclined surface parallel with the disc 610. The support column 621 is vertically connected to the loading surface of the bottom plate 620. The other end of the support column 621 is connected to a bottom of the disc 610. The support column 621 is configured to enhance the stability of the disc 610 and enhance the stability of the incubating tray 611 during operation.

In order to prevent the main spindle 62 from being interfered by the external environment during operation, the first driving component may further include a protective housing 67 sleeved outside the main spindle 62. A bearing 68 is provided between the protective housing 67 and the main spindle 62. One end of the protective housing 67 is connected to the bottom plate 620, the other end thereof extends towards the disc 610. The main spindle 62 is wrapped and protected by the protective housing 67 to improve the stability of the device during operation.

The liquid injection and suction module 70 includes: a frame 71, a liquid injection component 76 connected to the frame 71, and a liquid suction component 79 connected to the frame 71. The liquid injection component 76 includes a liquid injection fixing base 77 hinged on the frame 71 and a liquid injection needle 78 connected to the liquid injection fixing base 77. The liquid suction component 79 includes a liquid suction fixing base 710 slidably connected to the frame 71 and a liquid suction unit 711 connected to the liquid suction fixing base 710. The liquid suction unit 711 is provided with a liquid suction needle 712.

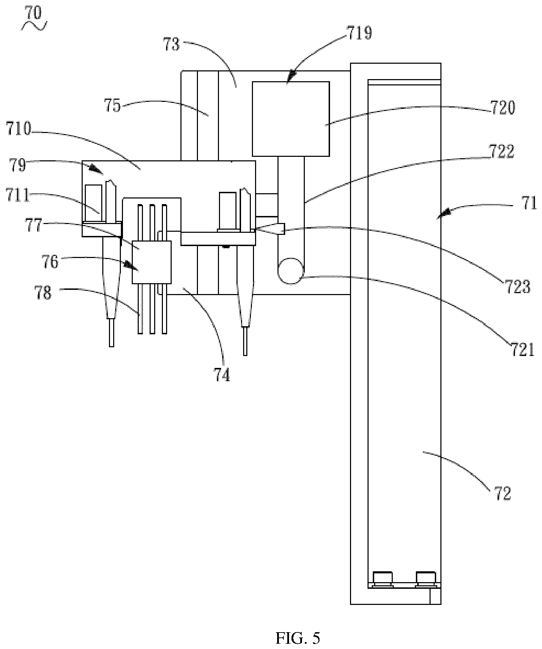

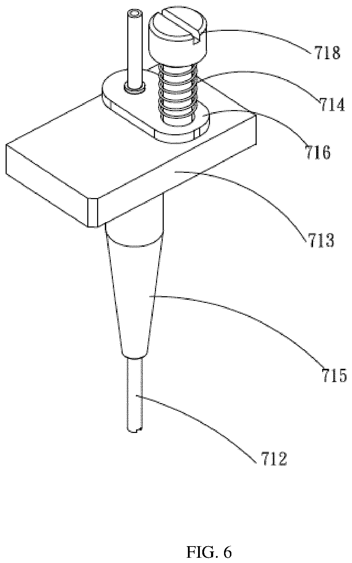

As shown in FIG. 5 and 6, the liquid injection and suction module 70 includes: a frame 71, a liquid injection component 76 connected to the frame 71, and a liquid suction component 79 connected to the frame 71. The liquid injection component 76 is configured for liquid injection, and the liquid suction component 79 is configured for liquid removal. In this embodiment, the frame 71 includes a support vertical plate 72 and a main fixing plate 73 connected to one side of the support vertical plate 72. The main fixing plate 73 is provided with a connecting plate 74 and a guiding rail 75 thereon. The connecting plate 74 is configured to be hinged to the liquid injection component 76, and the guiding rail 75 is arranged in a vertical direction and is slidably connected to the liquid suction component 79.

The liquid injection component 76 includes a liquid injection fixing base 77 hinged on the frame 71 and a liquid injection needle 78 connected to the liquid injection fixing base 77. In this embodiment, the liquid injection fixing base 77 is hinged on the connecting plate 74 by a hinge. The hinge manner allows the injection angle of the injection needle 78 to be adjusted in a vertical plane, and the injection angle can be adjusted according to the length of different samples. In other embodiments, an auxiliary sliding rail that is slidably connected to the connecting plate 74 may be provided on the main fixing plate 73. By sliding the connecting plate 74, the injection needle 78 can be raised and lowered relative to the frame 71, and the liquid injection needle 78 can be adjusted according to the process. In addition, the liquid injection needle 78 is a needle made of antirust metal with high finishing on internal and external surfaces, which effectively reduces the wall hanging liquid of the liquid injection needle 78 and improves the accuracy of liquid injection.

The liquid suction component 79 includes a liquid suction fixing base 710 slidably connected to the frame 71 and a liquid suction unit 711 connected to the liquid suction fixing base 710. As shown in FIG. 6, the liquid suction unit 711 includes: a liquid suction needle 712, a liquid suction plate 713 connected to the liquid suction fixing base 710, an elastic member 714 connected between the liquid suction plate 713 and the liquid suction needle 712, and a surface of the liquid suction plate 713 is sleeved with the support base 715 of the liquid suction needle 712. The liquid injection needle 78 penetrates the liquid suction plate 713 and extends through the support base 715, and the liquid suction needle 712 and the liquid suction plate 713 can slide relative to each other. The position of the liquid suction needle 712 can be adjusted according to operation requirements. The elastic sliding connection between the liquid suction plate 713 and the liquid suction needle 712 can be achieved by the elastic member 714, and the elastic member 714 can play a buffering effect when the liquid suction needle 712 abuts against the sample. The support base 715 is configured to limit the motion track of the liquid suction needle 712 and enhance the working stability of the liquid suction needle 712. In this embodiment, a baffle 716 is sleeved on an end of the liquid suction needle 712 away from the support base 715, and a surface of the liquid suction plate 713 facing away from the support base 715 is provided with a screw 718 arranged perpendicular to the liquid suction plate 713. The elastic member 714 is a buffer spring sleeved on the nut and is located between the head of the nut and the baffle 716 (in other embodiments, the elastic member 714 may also be an elastic plastic block or an elastic sheet). An end of the liquid suction needle 712 sleeved on the support base 715 is a needle end. When the needle end of the liquid suction needle 712 abuts against the sample, the liquid suction plate 713 can still be lowered by a certain distance under the action of the elastic member 714. The suction needle 712 then retracts upward, and at the same time, under the action of the elastic member 714, the tightness of the abutment between the needle end of the suction needle 712 and the sample is ensured, and the amount of residual liquid is reduced. In this embodiment, the number of the liquid suction units 711 is two and the two liquid suction units 711 are arranged at both ends of the liquid suction fixing base 710, respectively. During the liquid suction, the two liquid suction units 711 abut against both ends of the sample, respectively, so as to solve the problem of poor suction caused by poor fluidity on the left and right sides of a longer sample, and to ensure the residual liquid volume requirement of the sample.

In this embodiment, the liquid suction module 70 further includes a second driving component 719 connected to the liquid suction component 79. The second driving component 719 is connected to the liquid suction fixing base 710 to drive the liquid suction component 79 to slide to realize mechanized adjustment. The second driving component 719 includes: a second driver 720 connected to the frame 71, a driven pulley 721 connected to the frame 71, and a transmission unit 722 connected between the second driver 720 and the driven pulley 721. The transmission unit 722 is connected to the liquid suction fixing base 710. The rotation of the second driver 720 drives the transmission unit 722 to move, thereby driving the liquid suction component 79 to slide. In addition, in order to realize precise and automated operations, the second driving component 719 may further include a position detecting unit 723 connected to the second driver 720. The position detecting unit 723 is mounted on the base to detect the position of the liquid suction fixing base 710.

The determining module 80 includes: a camera facing the incubating tray 611, a light source disposed adjacent to the camera, and a determining component electrically connected to the camera. The determining component is configured to analyze the image and output the result.

The drying module 90 includes: a heater facing the incubating tray 611, a fan disposed adjacent to the heater, and a temperature sensor configured to detect a heating temperature.

In this embodiment, the full-automatic immunoblotting analyzer 10 further includes: a box 100 configured to accommodate the reagent module 20, the sample feeding module 30, the adding module 40, the washing module 50, the incubating module 60, the liquid injection and suction module 70, the determining module 80, and the drying module 90. The box 100 is provided with a protective cover 101 that can be turned over and opened.

In the aforementioned full-automatic immunoblotting analyzer 10, the incubating module 60 is taken as a core, and the other modules are configured around the incubating module 60. According to the testing items, the sample feeding module 30, the reagent module 20, and the adding module 40 are used to realize automatic sample feeding and automatic reagent adding, the washing module 50 is used to wash the adding module 40 to avoid contamination between the medicaments, the liquid injection and suction module 70 is used to inject the washing liquid to the incubating module 60 and discharge the waste liquid from the incubating module 60, the drying module 90 is used to dry the incubating module 60, and the determining module 80 is used to analyze the image of the incubating tray 611 and output the result. The full-automatic immunoblotting analyzer does not require human involvement in the test process, and the modules cooperate with each other to realize the whole process from sample feeding to result output.

Meanwhile, a full-automatic immunoblotting analysis testing method is further provided.

A full-automatic immunoblotting analysis testing method includes:

In step 1, a full-automatic immunoblotting analyzer 10 is provided, which includes: a reagent module 20, a sample feeding module 30, an adding module 40, a washing module 50, an incubating module 60, a liquid injection and suction module 70, a determining module 80 and a drying module 90. The reagent module 20 is configured to load a reagent required for testing. The sample feeding module 30 is configured to receive a sample and deliver the sample to a specified sampling station. The adding module 40 is configured to suck the reagent from the reagent module 20 and deliver the reagent to a specified reagent adding station, which is a testing slit 614 of the incubating module 60. The adding module 40 is further configured to suck the sample from the adding module 40 and deliver the sample to a specified sample adding station, which is the testing slit 614 of the incubating module 60. The washing module 50 is configured to wash the adding module 40. The incubating module 60 is configured to receive the reagent and the sample delivered by the adding module 40, and mix and incubate the reagent and the sample. The incubating module 60 is provided with a testing slit 614 configured to carry the reagent and the sample. The liquid injection and suction module 70 is provided at an edge of the incubating module 60 and is configured to inject washing liquid and suck liquid from the incubating module 60. The determining module 80 is disposed at the edge of the incubating module 60 for image shooting, image analysis, and result output of the substance in the incubating module 60. The drying module 90 is provided at the edge of the incubating module 60 and configured to dry the incubating module 60.

In step 2, amount of reagents are sucked from the reagent module 20 and amount of samples are sucked from the sample feeding module 30 by the adding module 40, and the reagents and the samples are delivered into the testing slit 614 of the incubating module 60 to perform an incubation operation by the adding module 40. The reagents have one or more types. Prior to sucking different reagents and samples, the adding module is washed by the washing module 50.

For step 2, the types of reagents will be adjusted according to different test items. Moreover, the order of adding different reagents and the order of adding samples are also adjusted adaptively according to different items. An example of a test item containing four reagents is described as follows:

In step 2.1, amount of first reagents are sucked by the adding module 40 from the reagent module 20 and transferred to the incubating module 60, the first reagents are released to all the testing slits 614 one by one.

In step 2.2, the incubating module 60 is rotated and incubated for a certain period of time, then the liquid in all of the testing slits 614 is drained one by one by the liquid injection and suction module 70.

In step 2.3, the adding module 40 is rotated to a washing station, and the adding module 40 is washed by the washing module 50.

In step 2.4, amount of second reagents are sucked by the adding module 40 from the reagent module 20 and transferred to the incubating module 60, the second reagents are released to all the testing slits 614 one by one.

In step 2.5, the adding module 40 is rotated to the washing station, and the adding module 40 is washed by the washing module 50.

In step 2.6, amount of samples are sucked by the adding module 40 from the sample feeding module 30 and transferred to the incubating module 60, the samples are released to all the testing slits 614 one by one.

In step 2.7, the incubating module 60 is rotated and incubated for a certain period of time, then the liquid in all of the testing slits 614 is drained one by one by the liquid injection and suction module 70.

In step 2.8, washing liquid is injected into all of the testing slits 614 one by one by the liquid injection and suction module 70.

In step 2.9, the incubating module 60 is rotated and incubated for a certain period of time, then the liquid in all of the testing slits 614 is drained one by one by the liquid injection and suction module 70.

In step 2.10, the adding module 40 is rotated to the washing station, and the adding module 40 is washed by the washing module 50.

In step 2.11, amount of third reagents are sucked by the adding module 40 from the reagent module 20, and transferred and released to the incubating module 60, the third reagents are released to all the testing slits 614 one by one.

In step 2.12, the incubating module 60 is rotated and incubated for a certain period of time, then the liquid in all of the testing slits 614 is drained one by one by the liquid injection and suction module 70.

In step 2.13, the adding module 40 is rotated to the washing station, and the adding module 40 is washed by the washing module 50.

In step 2.14, amount of fourth reagents are sucked by the adding module 40 from the reagent module 20 and transferred to the incubating module 60, the fourth reagents are released to all the testing slits 614 one by one.

In step 3, the incubating module 60 is rotated and incubated for a certain period of time, then the liquid in all of the testing slits 614 is drained one by one by the liquid injection and suction module 70.

In step 4, washing liquid is injected into all the testing slits 614 one by one by the liquid injection and suction module 70.

In step 5, the drying module 90 is activated, and the incubating module 60 is started to rotate for a predetermined time and then stopped.

In step 6, the determining module 80 is activated to identify objects in all of the testing slits 614 one by one.

In step 7, analysis result is outputted by the determining module 80.

It should be noted that the full-automatic immunoblotting analyzer 10 in the full-automatic immunoblotting analysis testing method of this embodiment can add features according to the aforementioned description of the device part, and will not be repeated here.

In the aforementioned full-automatic immunoblotting analysis testing method, the incubating module 60 is taken as a core, and the other modules are configured around the incubating module 60. According to the testing items, the sample feeding module 30, the reagent module 20, and the adding module 40 are used to realize automatic sample feeding and automatic reagent adding, the washing module 50 is used to wash the adding module 40 to avoid contamination between the medicaments, the liquid injection and suction module 70 is used to inject the washing liquid to the incubating module 60 and discharge the waste liquid from the incubating module 60, the drying module 90 is used to dry the incubating module 60, and the determining module 80 is used to analyze the image of the incubating tray 611 and output the result. The full-automatic immunoblotting analyzer does not require human involvement in the test process, and the modules cooperate with each other to realize the whole process from sample feeding to result output.

## Claims

1. A full-automatic immunoblotting analyzer (10), comprising:
a reagent module (20) configured to load a reagent required for testing;
a sample feeding module (30) configured to receive a sample and deliver the sample to a specified sampling station;
an adding module (40) configured to suck the reagent from the reagent module (20) and deliver the reagent to a testing slit (614) of an incubating module (60); wherein the adding module (40) is further configured to suck the sample from the sample feeding module (30), and deliver the sample to the testing slit (614) of the incubating module (60);
a washing module configured to wash the adding module (40);
the incubating module (60) configured to receive the reagent and the sample delivered by the adding module (40), and mix and incubate the reagent and the sample; wherein the incubating module (60) is provided with the testing slit (614) configured to carry the reagent and the sample;
a liquid injection and suction module (70) provided at an edge of the incubating module (60) and configured to inject washing liquid and suck liquid from the incubating module (60);
a determining module (80) provided at the edge of the incubating module (60) and configured to take an image of a substance in the incubating module (60) and analyze the image, and output a result; and
a drying module (90) provided at the edge of the incubating module (60) and configured to dry the incubating module (60);
wherein the incubating module (60) comprises a first driving component and a testing component (69) connected to the first driving component (61), the first driving component (61) comprises a first driver (63) and a main spindle (62) connected to the first driver (63), the first driver (63) is configured to drive the main spindle (62) to rotate, an angle between the main spindle (62) and a vertical direction is θ and θ is an acute angle, the testing component (69) comprises a disc (610) sleeved on the main spindle (62) and an incubating tray (611) located in the disc (610), the disc (610) is provided with a through hole allowing the main spindle (62) to extend through, the disc (610) is provided with a thermal insulation pot (612) to accommodate the incubating tray (611) and a heating film (613) connected to the thermal insulation pot (612), an angle between the incubating tray (611) and a horizontal plane is β and β=θ, the incubating tray (611) is connected to the main spindle (62) and rotates with the main spindle (62), the incubating tray (611) is provided with the testing slit (614) configured to carry the sample and the reagent.

2. The full-automatic immunoblotting analyzer (10) according to claim 1, wherein the sample feeding module (30) comprises: a first X-axis movement component, a first Y-axis movement component connected to the first X-axis movement component, and a first Z-axis movement component connected to the first Y-axis movement component, wherein the first X-axis movement component, the first Y-axis movement component, and the first Z-axis movement component cooperate with each other to realize a spatial three-dimensional action.

3. The full-automatic immunoblotting analyzer (10) according to claim 1, wherein the adding module (40) comprises a second Z-axis movement component and a horizontal θ-axis movement component connected to the second Z-axis movement component, wherein the second Z-axis movement component is configured to adjust height, and the horizontal θ-axis movement component is configured to realize horizontal position adjustment via rotating and swinging.

4. The full-automatic immunoblotting analyzer (10) according to claim 1, wherein the disc (610) is further provided with a pressing plate (616) and a thermal insulation cotton component, the pressing plate (616) is located at a bottom of the thermal insulation pot (612), and the heating film (613) is located between the thermal insulation pot (612) and the pressing plate (616), the thermal insulation cotton component is located outside the thermal insulation pot (612) to keep the thermal insulation pot (612) warm.

5. The full-automatic immunoblotting analyzer (10) according to claim 1, wherein the liquid injection and suction module (70) comprises: a frame (71), a liquid injecting component connected to the frame (71), and a liquid suction component (79) connected to the frame (71), the liquid suction component (79) comprises a liquid injection fixing base (77) hinged on the frame (71) and a liquid injection needle (78) connected to the liquid injection fixing base (77), the liquid suction component (79) comprises a liquid suction fixing base (710) slidably connected to the frame (71) and a liquid suction unit (711) connected to the liquid suction fixing base (710), the liquid suction unit (711) is provided with a liquid suction needle (712).

6. The full-automatic immunoblotting analyzer (10) according to claim 1, wherein the determining module (80) comprises: a camera facing the incubating tray (611), a light source disposed adjacent to the camera, and a determining component electrically connected to the camera, wherein the determining component is configured to analyze the image and output the result.

7. The full-automatic immunoblotting analyzer (10) according to claim 1, wherein the drying module (90) comprises: a heater facing the incubating tray (611), a fan disposed adjacent to the heater, and a temperature sensor configured to detect a heating temperature.

8. The full-automatic immunoblotting analyzer (10) according to any one of claims 1 to 7, further comprising: a box (100) configured to accommodate the reagent module (20), the sample feeding module (30), the adding module (40), the washing module, the incubating module (60), the liquid injection and suction module (70), the determining module (80), and the drying module (90).

9. A full-automatic immunoblotting analysis testing method, comprising:
providing a full-automatic immunoblotting analyzer (10) comprising: a reagent module (20), a sample feeding module (30), an adding module (40), a washing module (50), an incubating module (60), a liquid injection and suction module (70), a determining module (80), and a drying module (90); wherein the reagent module (20) is configured to load a reagent required for testing; the sample feeding module (30) is configured to receive a sample and deliver the sample to a specified sampling station; the adding module (40) is configured to suck the reagent from the reagent module (20) and deliver the reagent to a testing slit (614) of the incubating module (60); the adding module (40) is also configured to suck the sample from the sample feeding module (30), and deliver the sample to the testing slit (614) of the incubating module (60); the washing module (50) is configured to wash the adding module (40); the incubating module (60) is configured to receive the reagent and the sample sent from the adding module (40), and the reagent and the sample are mixed and incubated; the incubating module (60) is provided with the testing slit (614) configured to carry the reagent and the sample; the liquid injection and suction module (70) is provided at an edge of the incubating module (60) and configured to inject washing liquid and suck liquid from the incubating module (60); the determining module (80) is disposed at the edge of the incubation module for image shooting, image analysis, and result output of the substance in the incubation module; the drying module (90) is provided at the edge of the incubating module (60) and configured to dry the incubating module (60);
sucking, by the adding module (40), amount of reagents from the reagent module (20) and sucking amount of samples from the sample feeding module (30), and delivering, by the adding module (40), the reagents and the samples into the testing slit (614) of the incubating module (60) to perform an incubation operation, wherein the reagents have one or more types, and prior to sucking different reagents and samples, washing, by the washing module (50), the adding module (40);
rotating the incubating module (60) and incubating for a certain period of time, then draining, by the liquid injection and suction module (70), the liquid in all of the testing slits (614) one by one;
injecting, by the liquid injection and suction module (70), washing liquid into all of the testing slits (614) one by one;
activating the drying module (90), and starting the incubating module (60) to rotate for a predetermined time and then stopping;
activating the determining module (80) to identify objects in all of the testing slits (614) one by one; and
outputting, by the determining module (80), analysis result;
wherein the incubating module (60) comprises a first driving component and a testing component (69) connected to the first driving component (61), the first driving component (61) comprises a first driver (63) and a main spindle (62) connected to the first driver (63), the first driver (63) is configured to drive the main spindle (62) to rotate, an angle between the main spindle (62) and a vertical direction is θ and θ is an acute angle, the testing component (69) comprises a disc (610) sleeved on the main spindle (62) and an incubating tray (611) located in the disc (610), the disc (610) is provided with a through hole allowing the main spindle (62) to extend through, the disc (610) is provided with a thermal insulation pot (612) to accommodate the incubating tray (611) and a heating film (613) connected to the thermal insulation pot (612), an angle between the incubating tray (611) and a horizontal plane is β and β=θ, the incubating tray (611) is connected to the main spindle (62) and rotates with the main spindle (62).

## Patentansprüche

1. Vollautomatischer Immunblotting-Analysator (10), der Folgendes umfasst:
ein Reagenzmodul (20), das konfiguriert ist, um ein Reagenz zu laden, das zum Testen erforderlich ist;
ein Probenzuführungsmodul (30), das konfiguriert ist, um eine Probe zu empfangen und die Probe an eine bestimmte Probenentnahmestation zu liefern;
ein Hinzufügungsmodul (40), das konfiguriert ist, um das Reagenz aus dem Reagenzmodul (20) zu saugen und das Reagenz an einen Testschlitz (614) eines Inkubationsmoduls (60) zu liefern; wobei das Hinzufügungsmodul (40) ferner konfiguriert ist, um die Probe aus dem Probenzuführungsmodul (30) zu saugen und die Probe an den Testschlitz (614) des Inkubationsmoduls (60) zu liefern;
ein Waschmodul, das konfiguriert ist, um das Hinzufügungsmodul (40) zu waschen;
wobei das Inkubationsmodul (60) konfiguriert ist, um das Reagenz und die Probe zu empfangen, die von dem Hinzufügungsmodul (40) geliefert wird, und das Reagenz und die Probe zu mischen und zu inkubieren; wobei das Inkubationsmodul (60) mit dem Testschlitz (614) versehen ist, der konfiguriert ist, um das Reagenz und die Probe zu tragen;
ein Flüssigkeitseinspritzungs- und -saugmodul (70), das an einer Kante des Inkubationsmoduls (60) vorgesehen ist und konfiguriert ist, um Waschflüssigkeit einzuspritzen und Flüssigkeit aus dem Inkubationsmodul (60) zu saugen;
ein Bestimmungsmodul (80), das an der Kante des Inkubationsmoduls (60) vorgesehen ist und konfiguriert ist, um ein Bild einer Substanz in dem Inkubationsmodul (60) aufzunehmen und das Bild zu analysieren und ein Ergebnis auszugeben; und
ein Trocknungsmodul (90), das an der Kante des Inkubationsmoduls (60) vorgesehen ist und konfiguriert ist, um das Inkubationsmodul (60) zu trocknen;
wobei das Inkubationsmodul (60) eine erste Antriebskomponente und eine Testkomponente (69) umfasst, die mit der ersten Antriebskomponente (61) verbunden ist, wobei die erste Antriebskomponente (61) einen ersten Antrieb (63) und eine Hauptspindel (62) umfasst, die mit dem ersten Antrieb (63) verbunden ist, wobei der erste Antrieb (63) konfiguriert ist, um die Hauptspindel (62) zum Drehen anzutreiben, wobei ein Winkel zwischen der Hauptspindel (62) und einer vertikalen Richtung θ ist und θ ein spitzer Winkel ist, wobei die Testkomponente (69) eine Scheibe (610), die auf der Hauptspindel (62) gelagert ist, und eine Inkubationsschale (611) umfasst, die in der Scheibe (610) angeordnet ist, wobei die Scheibe (610) mit einem Durchgangsloch versehen ist, das der Hauptspindel (62) ermöglicht, sich dadurch zu erstrecken, wobei die Scheibe (610) mit einem Wärmeisolationstopf (612) zur Aufnahme der Inkubationsschale (611) und einer mit dem Wärmeisolationstopf (612) verbundenen Heizfolie (613) versehen ist, wobei ein Winkel zwischen der Inkubationsschale (611) und einer horizontalen Ebene β ist und β=θ ist, wobei die Inkubationsschale (611) mit der Hauptspindel (62) verbunden ist und sich mit der Hauptspindel (62) dreht, wobei die Inkubationsschale (611) mit dem Testschlitz (614) versehen ist, der konfiguriert ist, um die Probe und das Reagenz zu tragen.

2. Vollautomatischer Immunblotting-Analysator (10) nach Anspruch 1, wobei das Probenzuführungsmodul (30) Folgendes umfasst: eine erste X-Achsen-Bewegungskomponente, eine erste Y-Achsen-Bewegungskomponente, die mit der ersten X-Achsen-Bewegungskomponente verbunden ist, und eine erste Z-Achsen-Bewegungskomponente, die mit der ersten Y-Achsen-Bewegungskomponente verbunden ist, wobei die erste X-Achsen-Bewegungskomponente, die erste Y-Achsen-Bewegungskomponente und die erste Z-Achsen-Bewegungskomponente miteinander kooperieren, um eine räumliche dreidimensionale Aktion zu realisieren.

3. Vollautomatischer Immunblotting-Analysator (10) nach Anspruch 1, wobei das Hinzufügungsmodul (40) eine zweite Z-Achsen-Bewegungskomponente und eine horizontale θ-Achsen-Bewegungskomponente umfasst, die mit der zweiten Z-Achsen-Bewegungskomponente verbunden ist, wobei die zweite Z-Achsen-Bewegungskomponente konfiguriert ist, um die Höhe einzustellen, und die horizontale θ-Achsen-Bewegungskomponente konfiguriert ist, um eine horizontale Positionseinstellung durch Drehen und Schwingen zu realisieren.

4. Vollautomatischer Immunblotting-Analysator (10) nach Anspruch 1, wobei die Scheibe (610) ferner mit einer Pressplatte (616) und einer wärmeisolierenden Baumwollkomponente versehen ist, wobei die Pressplatte (616) an einem Boden des Wärmeisolationstopfs (612) angeordnet ist und die Heizfolie (613) zwischen dem Wärmeisolationstopf (612) und der Pressplatte (616) angeordnet ist, wobei die wärmeisolierende Baumwollkomponente außerhalb des Wärmeisolationstopfs (612) angeordnet ist, um den Wärmeisolationstopf (612) warm zu halten.

5. Vollautomatischer Immunblotting-Analysator (10) nach Anspruch 1, wobei das Flüssigkeitseinspritzungs- und -saugmodul (70) Folgendes umfasst: einen Rahmen (71), eine Flüssigkeitseinspritzungskomponente, die mit dem Rahmen (71) verbunden ist, und eine Flüssigkeitssaugkomponente (79), die mit dem Rahmen (71) verbunden ist, wobei die Flüssigkeitssaugkomponente (79) eine Flüssigkeitseinspritzungsbefestigungsbasis (77), die an dem Rahmen (71) angelenkt ist, und eine Flüssigkeitseinspritzungsnadel (78) umfasst, die mit der Flüssigkeitseinspritzungsbefestigungsbasis (77) verbunden ist, wobei die Flüssigkeitssaugkomponente (79) eine Flüssigkeitssaugbefestigungsbasis (710), die mit dem Rahmen (71) verschiebbar verbunden ist, und eine Flüssigkeitssaugeinheit (711) umfasst, die mit der Flüssigkeitssaugbefestigungsbasis (710) verbunden ist, wobei die Flüssigkeitssaugeinheit (711) mit einer Flüssigkeitssaugnadel (712) versehen ist.

6. Vollautomatischer Immunblotting-Analysator (10) nach Anspruch 1, wobei das Bestimmungsmodul (80) Folgendes umfasst: eine Kamera, die der Inkubationsschale (611) zugewandt ist, eine Lichtquelle, die neben der Kamera angeordnet ist, und eine Bestimmungskomponente, die mit der Kamera elektrisch verbunden ist, wobei die Bestimmungskomponente konfiguriert ist, um das Bild zu analysieren und das Ergebnis auszugeben.

7. Vollautomatischer Immunblotting-Analysator (10) nach Anspruch 1, wobei das Trocknungsmodul (90) Folgendes umfasst: eine Heizvorrichtung, die der Inkubationsschale (611) zugewandt ist, ein Gebläse, das neben der Heizvorrichtung angeordnet ist, und einen Temperatursensor, der konfiguriert ist, um eine Heiztemperatur zu erkennen.

8. Vollautomatischer Immunblotting-Analysator (10) nach einem der Ansprüche 1 bis 7, der ferner Folgendes umfasst: einen Kasten (100), der konfiguriert ist, um das Reagenzmodul (20), das Probenzuführungsmodul (30), das Hinzufügungsmodul (40), das Waschmodul, das Inkubationsmodul (60), das Flüssigkeitseinspritzungs- und -saugmodul (70), das Bestimmungsmodul (80) und das Trocknungsmodul (90) aufzunehmen.

9. Vollautomatische Testmethode für die Immunblotting-Analyse, die Folgendes umfasst:
Bereitstellen eines vollautomatischen Immunblotting-Analysators (10), der Folgendes umfasst: ein Reagenzmodul (20), ein Probenzuführungsmodul (30), ein Hinzufügungsmodul (40), ein Waschmodul (50), ein Inkubationsmodul (60), ein Flüssigkeitseinspritzungs- und -saugmodul (70), ein Bestimmungsmodul (80) und ein Trocknungsmodul (90); wobei das Reagenzmodul (20) konfiguriert ist, um ein zum Testen erforderliches Reagenz zu laden; wobei das Probenzuführungsmodul (30) konfiguriert ist, um eine Probe zu empfangen und die Probe an eine bestimmte Probenentnahmestation zu liefern; wobei das Hinzufügungsmodul (40) konfiguriert ist, um das Reagenz aus dem Reagenzmodul (20) zu saugen und das Reagenz an einen Testschlitz (614) des Inkubationsmoduls (60) zu liefern; wobei das Hinzufügungsmodul (40) auch konfiguriert ist, um die Probe aus dem Probenzuführungsmodul (30) zu saugen und die Probe an den Testschlitz (614) des Inkubationsmoduls (60) zu liefern; wobei das Waschmodul (50) konfiguriert ist, um das Hinzufügungsmodul (40) zu waschen; wobei das Inkubationsmodul (60) konfiguriert ist, um das Reagenz und die Probe zu empfangen, die von dem Hinzufügungsmodul (40) gesendet wird, und das Reagenz und die Probe vermischt und inkubiert werden; wobei das Inkubationsmodul (60) mit dem Testschlitz (614) versehen ist, der konfiguriert ist, um das Reagenz und die Probe zu tragen; wobei das Flüssigkeitseinspritzungs- und -saugmodul (70) an einer Kante des Inkubationsmoduls (60) vorgesehen ist und konfiguriert ist, um Waschflüssigkeit einzuspritzen und Flüssigkeit aus dem Inkubationsmodul (60) zu saugen; wobei das Bestimmungsmodul (80) an der Kante des Inkubationsmoduls zur Bildaufnahme, Bildanalyse und Ergebnisausgabe der Substanz in dem Inkubationsmodul angeordnet ist; wobei das Trocknungsmodul (90) an der Kante des Inkubationsmoduls (60) vorgesehen ist und konfiguriert ist, um das Inkubationsmodul (60) zu trocknen; wobei durch das Hinzufügungsmodul (40) eine Menge an Reagenzien aus dem Reagenzmodul (20) gesaugt wird und eine Menge an Proben aus dem Probenzuführungsmodul (30) gesaugt wird, und wobei die Reagenzien und
die Proben durch das Hinzufügungsmodul (40) in den Testschlitz (614) des Inkubationsmoduls (60) geliefert werden, um einen Inkubationsvorgang auszuführen, wobei die Reagenzien einen oder mehrere Typen haben und das Hinzufügungsmodul (40) vor dem Saugen verschiedener Reagenzien und Proben durch das Waschmodul (50) gewaschen wird;
Drehen des Inkubationsmoduls (60) und Inkubieren für eine bestimmte Zeitdauer, dann Ablassen der Flüssigkeit in allen Testschlitzen (614) nacheinander durch das Flüssigkeitseinspritzungs- und -saugmodul (70);
Einspritzen von Waschflüssigkeit durch das Flüssigkeitseinspritzungs- und - saugmodul (70) nacheinander in alle Testschlitze (614);
Aktivieren des Trocknungsmoduls (90) und Starten des Inkubationsmoduls (60), um sich für eine vorbestimmte Zeit zu drehen und dann anzuhalten;
Aktivieren des Bestimmungsmoduls (80), um Objekte in allen Testschlitzen (614) nacheinander zu identifizieren; und
Ausgeben eines Analyseergebnisses durch das Bestimmungsmodul (80);
wobei das Inkubationsmodul (60) eine erste Antriebskomponente und eine Testkomponente (69) umfasst, die mit der ersten Antriebskomponente (61) verbunden ist, wobei die erste Antriebskomponente (61) einen ersten Antrieb (63) und eine Hauptspindel (62) umfasst, die mit dem ersten Antrieb (63) verbunden ist, wobei der erste Antrieb (63) konfiguriert ist, um die Hauptspindel (62) zum Drehen anzutreiben, wobei ein Winkel zwischen der Hauptspindel (62) und einer vertikalen Richtung θ ist und θ ein spitzer Winkel ist, wobei die Testkomponente (69) eine Scheibe (610), die auf der Hauptspindel (62) gelagert ist, und eine Inkubationsschale (611) umfasst, die in der Scheibe (610) angeordnet ist, wobei die Scheibe (610) mit einem Durchgangsloch versehen ist, das der Hauptspindel (62) ermöglicht, sich dadurch zu erstrecken, wobei die Scheibe (610) mit einem Wärmeisolationstopf (612) zum Aufnehmen der Inkubationsschale (611) und einer mit dem Wärmeisolationstopf (612) verbundenen Heizfolie (613) versehen ist, wobei ein Winkel zwischen der Inkubationsschale (611) und einer horizontalen Ebene β ist und β=θ ist, wobei die Inkubationsschale (611) mit der Hauptspindel (62) verbunden ist und sich mit der Hauptspindel (62) dreht.

## Revendications

1. Analyseur d'immunotransfert entièrement automatique (10), comprenant :
un module de réactif (20) configuré pour charger un réactif requis pour tester ;
un module d'alimentation en échantillon (30) configuré pour recevoir un échantillon et délivrer l'échantillon à une station d'échantillonnage spécifiée ;
un module d'ajout (40) configuré pour aspirer le réactif du module de réactif (20) et délivrer le réactif à une fente de test (614) d'un module d'incubation (60) ; où le module d'ajout (40) est en outre configuré pour aspirer l'échantillon du module d'alimentation en échantillon (30) et délivrer l'échantillon à la fente de test (614) du module d'incubation (60) ;
un module de lavage configuré pour laver le module d'ajout (40) ;
le module d'incubation (60) configuré pour recevoir le réactif et l'échantillon délivrés par le module d'ajout (40), et mélanger et incuber le réactif et l'échantillon ; où le module d'incubation (60) est pourvu de la fente de test (614) configurée pour transporter le réactif et l'échantillon ;
un module d'injection et d'aspiration de liquide (70) pourvu à un bord du module d'incubation (60) et configuré pour injecter du liquide de lavage et aspirer du liquide du module d'incubation (60) ;
un module de détermination (80) pourvu au bord du module d'incubation (60) et configuré pour prendre une image d'une substance dans le module d'incubation (60) et analyser l'image, et émettre un résultat ; et
un module de séchage (90) pourvu au bord du module d'incubation (60) et configuré pour sécher le module d'incubation (60) ;
où le module d'incubation (60) comprend un premier composant d'entraînement et un composant de test (69) connecté au premier composant d'entraînement (61), le premier composant d'entraînement (61) comprend un premier pilote (63) et une broche principale (62) connectée au premier pilote (63), le premier pilote (63) est configuré pour entraîner la broche principale (62) en rotation, un angle entre la broche principale (62) et une direction verticale est θ et θ est un angle aigu, le composant de test (69) comprend un disque (610) emmanché sur la broche principale (62) et un plateau d'incubation (611) situé dans le disque (610), le disque (610) est pourvu d'un trou traversant permettant à la broche principale (62) de s'étendre à travers, le disque (610) est pourvu d'un pot d'isolation thermique (612) pour accueillir le plateau d'incubation (611) et d'un film chauffant (613) connecté au pot d'isolation thermique (612), un angle entre le plateau d'incubation (611) et un plan horizontal est β et β = θ, le plateau d'incubation (611) est connecté à la broche principale (62) et tourne avec la broche principale (62), le plateau d'incubation (611) est pourvu avec la fente de test (614) configurée pour transporter l'échantillon et le réactif.

2. Analyseur d'immunotransfert entièrement automatique (10) selon la revendication 1, où le module d'alimentation en échantillon (30) comprend : un premier composant de mouvement de l'axe X, un premier composant de mouvement de l'axe Y connecté au premier composant de mouvement de l'axe X, et un premier composant de mouvement de l'axe Z connecté au premier composant de mouvement de l'axe Y, où le premier composant de mouvement de l'axe X, le premier composant de mouvement de l'axe Y et le premier composant de mouvement de l'axe Z coopèrent l'un avec l'autre pour réaliser une action spatiale tridimensionnelle.

3. Analyseur d'immunotransfert entièrement automatique (10) selon la revendication 1, où le module d'ajout (40) comprend un deuxième composant de mouvement de l'axe Z et un composant de mouvement de l'axe θ horizontal connecté au deuxième composant de mouvement de l'axe Z, où le deuxième composant de mouvement de l'axe Z est configuré pour ajuster la hauteur, et le composant de mouvement de l'axe θ horizontal est configuré pour réaliser un ajustement de position horizontale via rotation et oscillation.

4. Analyseur d'immunotransfert entièrement automatique (10) selon la revendication 1, où le disque (610) est en outre pourvu d'une plaque de pressage (616) et d'un composant en coton d'isolation thermique, la plaque de pressage (616) est située à un fond du pot d'isolation thermique (612), et le film chauffant (613) est situé entre le pot d'isolation thermique (612) et la plaque de pressage (616), le composant en coton d'isolation thermique est situé à l'extérieur du pot d'isolation thermique (612) pour maintenir le pot d'isolation thermique (612) chaud.

5. Analyseur d'immunotransfert entièrement automatique (10) selon la revendication 1, où le module d'injection et d'aspiration de liquide (70) comprend : un cadre (71), un composant d'injection de liquide connecté au cadre (71) et un composant d'aspiration de liquide (79) connecté au cadre (71), le composant d'aspiration de liquide (79) comprend une base de fixation d'injection de liquide (77) articulée sur le cadre (71) et une aiguille d'injection de liquide (78) connectée à la base de fixation d'injection de liquide (77), le composant d'aspiration de liquide (79) comprend une base de fixation d'aspiration de liquide (710) connectée de manière coulissante au cadre (71) et une unité d'aspiration de liquide (711) connectée à la base de fixation d'aspiration de liquide (710), l'unité d'aspiration de liquide (711) est pourvue d'une aiguille d'aspiration de liquide (712).

6. Analyseur d'immunotransfert entièrement automatique (10) selon la revendication 1, où le module de détermination (80) comprend : une caméra faisant face au plateau d'incubation (611), une source de lumière disposée adjacente à la caméra, et un composant de détermination connecté électriquement à la caméra, où le composant de détermination est configuré pour analyser l'image et émettre le résultat.

7. Analyseur d'immunotransfert entièrement automatique (10) selon la revendication 1, où le module de séchage (90) comprend : un chauffage faisant face au plateau d'incubation (611), un ventilateur disposé adjacent au chauffage, et un capteur de température configuré pour détecter une température de chauffage.

8. Analyseur d'immunotransfert entièrement automatique (10) selon l'une quelconque des revendications 1 à 7, comprenant en outre : une boîte (100) configurée pour accueillir le module de réactif (20), le module d'alimentation en échantillon (30), le module d'ajout (40), le module de lavage, le module d'incubation (60), le module d'injection et d'aspiration de liquide (70), le module de détermination (80) et le module de séchage (90).

9. Procédé de test d'analyse par immunotransfert entièrement automatique, comprenant :
fournir un analyseur d'immunotransfert entièrement automatique (10) comprenant : un module de réactif (20), un module d'alimentation en échantillon (30), un module d'ajout (40), un module de lavage (50), un module d'incubation (60), un module d'injection et d'aspiration de liquide (70), un module de détermination (80) et un module de séchage (90) ; où le module de réactif (20) est configuré pour charger un réactif requis pour le test ; le module d'alimentation en échantillon (30) est configuré pour recevoir un échantillon et délivrer l'échantillon à une station d'échantillonnage spécifiée ; le module d'ajout (40) est configuré pour aspirer le réactif du module de réactif (20) et délivrer le réactif à une fente de test (614) du module d'incubation (60) ; le module d'ajout (40) est également configuré pour aspirer l'échantillon du module d'alimentation en échantillon (30) et délivrer l'échantillon à la fente de test (614) du module d'incubation (60) ; le module de lavage (50) est configuré pour laver le module d'ajout (40) ; le module d'incubation (60) est configuré pour recevoir le réactif et l'échantillon envoyés du module d'ajout (40), et le réactif et l'échantillon sont mélangés et incubés ; le module d'incubation (60) est pourvu de la fente de test (614) configurée pour transporter le réactif et l'échantillon ; le module d'injection et d'aspiration de liquide (70) est pourvu à un bord du module d'incubation (60) et configuré pour injecter du liquide de lavage et aspirer du liquide du module d'incubation (60) ; le module de détermination (80) est disposé au bord du module d'incubation pour la prise d'image, l'analyse d'image et l'émission de résultat de la substance dans le module d'incubation ; le module de séchage (90) est pourvu au bord du module d'incubation (60) et configuré pour sécher le module d'incubation (60) ; aspirer, par le module d'ajout (40), une quantité de réactifs du module de réactifs (20) et aspirer une quantité d'échantillons du module d'alimentation en échantillon (30), et délivrer, par le module d'ajout (40), les réactifs et les échantillons dans la fente de test (614) du module d'incubation (60) pour effectuer une opération d'incubation, où les réactifs ont un ou plusieurs types, et avant d'aspirer différents réactifs et échantillons, laver, par le module de lavage (50), le module d'ajout (40) ;
faire tourner le module d'incubation (60) et incuber pour une certaine période de temps, puis drainer, par le module d'injection et d'aspiration de liquide (70), le liquide dans toutes les fentes de test (614) une par une ;
injecter, par le module d'injection et d'aspiration de liquide (70), du liquide de lavage dans toutes les fentes de test (614) une par une ;
activer le module de séchage (90), et commencer à faire tourner le module d'incubation (60) pour une durée prédéterminée et puis l'arrêter ;
activer le module de détermination (80) pour identifier des objets dans toutes les fentes de test (614) une par une ; et
émettre, par le module de détermination (80), un résultat d'analyse ;
où le module d'incubation (60) comprend un premier composant d'entraînement et un composant de test (69) connecté au premier composant d'entraînement (61), le premier composant d'entraînement (61) comprend un premier pilote (63) et une broche principale (62) connectée au premier pilote (63), le premier pilote (63) est configuré pour entraîner la broche principale (62) en rotation, un angle entre la broche principale (62) et une direction verticale est θ et θ est un angle aigu, le composant de test (69) comprend un disque (610) emmanché sur la broche principale (62) et un plateau d'incubation (611) situé dans le disque (610), le disque (610) est pourvu d'un trou traversant permettant à la broche principale (62) de s'étendre à travers, le disque (610) est pourvu d'un pot d'isolation thermique (612) pour accueillir le plateau d'incubation (611) et d'un film chauffant (613) connecté au pot d'isolation thermique (612), un angle entre le plateau d'incubation (611) et un plan horizontal est β et β = θ, le plateau d'incubation (611) est connecté à la broche principale (62) et tourne avec la broche principale.
